# EUROPEAN PATENT APPLICATION

(11) **EP 3 824 873 A1**
(43) Date of publication of application: **26.05.2021**
(21) Application number: 19211119.3
(22) Date of filing: 25.11.2019
(51) Int. Cl.: A61K 8/60, A61K 8/02, A61Q 11/00, A61C 3/025, B24C 7/00, B24C 11/00

(54) **USE OF RHAMNOSE AND ARABINOSE FOR DENTAL POWDER JET CLEANING**

(71) Applicant: Ferton Holding S.A., 2800 Delémont (CH)
(72) Inventor: Donnet, Marcel, 01630 Saint Jean de Gonville (FR); Gatti, Simone, 1022 Chavannes-près-Renens (CH)
(74) Representative: Müller Schupfner & Partner Patent- und Rechtsanwaltspartnerschaft mbB

(57) **Abstract**

The invention relates to the use of a powder in a powder jet device for cleaning tooth surfaces by powder spraying, wherein the powder comprises rhamnose and/or arabinose. The invention also relates to a process for cleaning tooth surfaces with a powder jet device, wherein a powder comprising rhamnose and/or arabinose is used.

## Description

The invention relates to the use of a powder in a powder jet device for cleaning tooth surfaces by powder spraying. The invention further relates to a process for cleaning teeth by using the powder in a powder jet device, wherein the powder is sprayed onto a tooth surface together with a gaseous and/or liquid carrier medium.

Professional dental prophylaxis is a maintenance treatment comprising powder jet cleaning or air polishing and aims to remove dental plaque and calculus that a patient is not able to remove during daily home care. The method is particularly effective since it allows to reach and clean all teeth surfaces and the interspaces between teeth, implants, brackets and appliances.

During powder jet cleaning, a powder is sprayed together with a gaseous carrier medium, usually air, onto tooth surfaces, thereby achieving an efficient cleaning. Additionally or as an alternative to a gaseous carrier medium, a liquid carrier medium, for example water may be used. Powder jet cleaning is performed with a powder jet device and it is particularly effective since it does not require repetitive movements nor different stages. Further, it is faster than other cleaning methods and it needs relatively low training to be learned correctly. The cleaning occurs without damaging enamel dentine and soft tissues as long as the powder is sufficiently soft and the particles are sufficiently small.

DE 200 09 665 U1 discloses a dental cleaning powder containing a basic powder of sodium bicarbonate (sodium hydrogencarbonate), alternatively calcium carbonate or glycine and additional active ingredients such as anti-microbial compounds or ingredients which contribute to the remineralisation of the teeth.

A powder for use in a powder jet device based on alditols, in particular mannitol and/or erythritol is described in EP 2 228 175 A1.

EP 3 253 359 A1 discloses that the particles should have an average particle size of around 10 - 100 µm for gentle and nevertheless efficient tooth cleaning. Small particles sizes are preferred for cleaning subgingival tooth surfaces and larger particle sizes are preferred for cleaning the supragingival tooth surfaces. Larger particle sizes usually result in discomfort or pain when subgingival tooth surfaces or soft tissues as gum are hit.

Important features of powders for use in dental cleaning are the hardness and the abrasiveness of the powders. A too hard and abrasive powder can damage the enamel and the composites used for dental reparation. Therefore, soft materials such as sodium bicarbonate are often used in powder jet devices. However, even with this material, patients often have discomfort and there is a risk of damaging soft tissues such as dentine and gingiva so that this technique was usually used only on enamel and in general supragingival.

Powders that were developed for subgingival treatment are for example the product PERIO from EMS (Electro Medical Systems GmbH) that is based on glycine. Such glycine powders are less abrasive and thus safer and more comfortable for patients even when used on soft tissues such as gingiva and exposed dentine. However, a high decrease in cleaning efficiency is usually experienced, compared with the more abrasive bicarbonate powders so that a higher amount of time and powder consumption is needed for the same treatment result.

A further powder that has therefore been developed is the product PLUS from EMS, based on erythritol, which is a material harder than glycine but softer than bicarbonate. The erythritol powder PLUS is more efficient in powder jet cleaning than glycine and thus a lower time and lower amount of powder is required to perform a treatment. However, the abrasiveness is again slightly enhanced.

In view of the above there is still a need for dental cleaning powder with lower abrasiveness and still high cleaning efficiency.

It is an object of the present invention to provide a powder for use in a powder jet device for cleaning tooth surfaces that overcome the afore-mentioned disadvantages of conventional powders, in particular a powder with lower abrasiveness than known powders and still high or even improved cleaning efficiency.

This object is achieved according to the invention by the use of a powder in a powder jet device for cleaning tooth surfaces by powder spraying, wherein the powder comprises rhamnose and/or arabinose. The object is further achieved by a process for cleaning tooth surfaces, wherein a powder comprising rhamnose and/or arabinose is sprayed with a powder jet device onto a tooth surface together with a gaseous and/or liquid carrier medium. The object is also achieved by a powder for use in a powder jet device for cleaning tooth surfaces by powder spraying, wherein the powder comprises rhamnose and/or arabinose.

Preferred embodiments of the invention are subject to the dependent claims as well as the following description.

It has surprisingly been found that rhamnose and arabinose powders provide a lower abrasiveness and simultaneously a higher cleaning efficiency than the common commercially available dental cleaning powders based on sodium bicarbonate or erythritol.

Cleaning tooth surfaces within the meaning of the invention is the partial or complete removal of dental plaque and/or calculus from tooth surfaces. The cleaning is performed with conventional powder jet devices for cleaning teeth by powder spraying as described above.

For an efficient tooth cleaning, the maximum particle size of the powder according to the invention (d₁₀₀) is preferably equal or less than 200 µm, more preferably ≤ 150 µm, even more preferably ≤ 120 µm and most preferably ≤ 100 µm. The average particle size (dso) is preferably 5 to 75 µm, in particular 10 to 70 µm. The particle sizes may be adapted to the field of application of dental cleaning that is provided. For example, a smaller average particle size of the particles is preferred for cleaning subgingival tooth surfaces, in particular about 5 to 40 µm, more preferably 10 to 30 µm. For cleaning supragingival tooth surfaces, a larger average particle size is preferred, in particular about 20 to 75 µm, more preferably 30 to 60 µm.

In the context of this invention, the d₅₀ value is the particle size at which 50 % of the particles are smaller than the d₅₀ value in terms of volume and 50 % of the particles are larger than the d₅₀ value in terms of volume. This applies also to d₁₀, d₉₀ and d₁₀₀ values. For a given d₉₀ value, 90 % of the particles with regard to the volume are smaller than this value. The maximum particle size is the d₁₀₀ value, where 100 % of the particles in terms of volume are smaller than this value. A powder with a certain maximum particle size can be prepared by sieving the powder with a sieve having an appropriate mesh size and the d₁₀₀ value can afterwards be determined by laser diffraction measurement. The d-values according to the invention are determined by laser diffraction using a dry dispersion unit (Malvern Mastersizer 2000, equipped with a Scirocco dry dispersion unit, operated at 1.5 bar). The measurement method is further described in the experimental section.

In a preferred embodiment of the invention, the powder comprising rhamnose and/or arabinose additionally comprises a flow aid, a bleaching agent, a bactericide, an analgesic and/or a flavouring agent. The total amount of these additional substances is preferably 0.3 to 5 wt.-%, based on the total weight of the powder, more preferably 0.5 to 2 wt.-%.

In a preferred embodiment of the invention, the powder comprises a flow aid, in particular a flow aid selected from the group consisting of silicon dioxide (silica), aluminium silicate and/or aluminium hydroxide. Silicon dioxide is most preferred, in particular in an amount of 0.5 to 2 wt.-%, based on the total weight of the powder.

In a further preferred embodiment of the invention, the powder comprises at least 50 wt.-% rhamnose and/or arabinose, more preferably at least 60 wt.-%, even more preferably 60 to 99.5 wt.-%, and most preferably 90 to 99.5 wt.-% rhamnose and/or arabinose.

Within the meaning of the present invention it should be understood that the amounts of the components of the powder, given in wt.-% (weight-%), sum up to 100 %. By way of example, a powder comprising 2 wt.-% amorphous silicon dioxide contains other components in a total amount of 98 wt.-%.

It is also preferred that the advantageous abrasiveness and efficiency of rhamnose and arabinose is combined with other and often cheaper cleaning powder components such a glycine, sodium hydrogen carbonate and/or alditol powders, preferably erythritol powder. A preferred powder of the invention comprises 40 to 80 wt.-% rhamnose and/or arabinose and 20 to 60 wt.-% glycine, sodium hydrogen carbonate and/or an alditol, preferably erythritol. The powder composition can additionally comprise other components as described herein, for example a flow aid, a bactericide, a bleaching agent and/or an analgesic.

The alditol according to the invention is preferably selected from the group consisting of erythritol, sorbitol, xylit (xylitol), mannit (mannitol), lactitol, threit (threitol), arabitol, and isomalt. Preferred bleaching agents are peroxides such as magnesium, calcium or zinc peroxides, persulfates such as sodium, potassium or ammonium persulfates or perborates.

Preferred bactericides are chlorhexidine, chlorhexidine digluconate, triclosan, cetylpyridinium chloride, hexetidine, tin(II) salts and zinc salts. Preferred analgesics are lidocaine and articaine.

It is also preferred that the powder for use in a powder jet device according to the invention consists of the described components. By way of example, a preferred powder consists of 98 - 99.5 wt.-% rhamnose and 0.5 - 2 wt.-% of a flow aid, in particular 0.5 - 2 wt.-% amorphous silicon dioxide, and optionally a bactericide, a bleaching agent and/or an analgesic. Another preferred powder consists of 98 - 99.5 wt.-% arabinose and 0.5 - 2 wt.-% of a flow aid, in particular 0.5 - 2 wt.-% amorphous silicon dioxide, and optionally a bactericide, a bleaching agent and/or an analgesic.

The invention also relates to a powder or powder mixture for use in a powder jet device for cleaning tooth surfaces by powder spraying. The preferred embodiments of the use described herein are also preferred for the powder or powder mixture. Each preferred, more and most preferred use embodiment described herein is also applicable to (a) powders that comprise rhamnose, but no arabinose, (b) powders that comprise arabinose, but no rhamnose, and (c) powders that comprise both rhamnose and arabinose.

The invention further relates to a process for cleaning tooth surfaces wherein the powder according to the invention is sprayed with a powder jet device onto the tooth surfaces together with a gaseous carrier medium and/or a liquid carrier medium, in particular air and optionally a fluid such as water.

The following examples provide preferred embodiments according to the invention.

### Examples

### Example 1: Rhamnose dental cleaning powder

Rhamnose was grinded and sieved at 120 µm, so that d₁₀₀ was ≤ 120 µm. The powder was mixed with about 1 wt.-% amorphous silicon dioxide (silica). The average particle size of this powder was 23 µm.

### Example 2: Arabinose dental cleaning powder

Arabinose was grinded and sieved at 120 µm, so that d₁₀₀ was ≤ 120 µm. The powder was mixed with about 1 wt.-% amorphous silicon dioxide (silica). The average particle size dso of this powder was 40 µm.

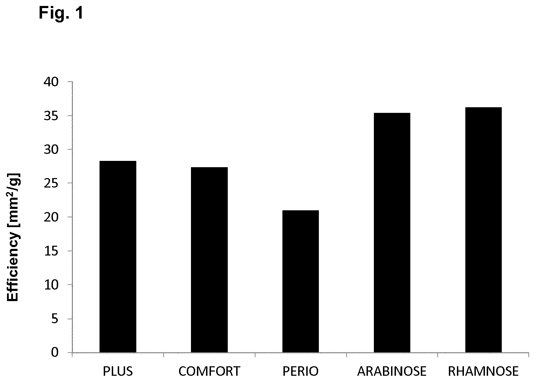

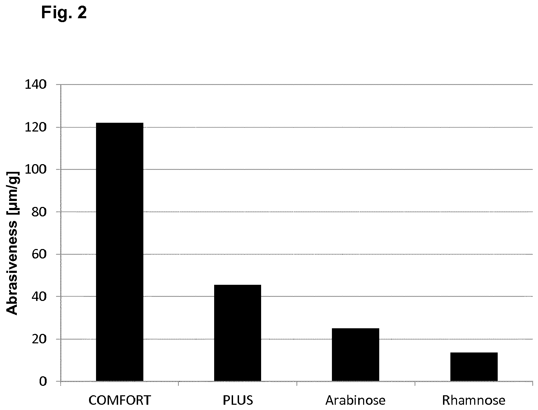

The two powders according to Example 1 and Example 2 were tested regarding cleaning efficiency and abrasiveness. The results are shown in Fig. 1 and 2 in comparison to known cleaning powders.

The efficiency or cleaning efficiency is the surface that is cleaned per gram of the powder. The abrasiveness is determined as follows:
Abrasiveness is tested by projecting powder with a powder jet device directly on a surface at 45° and 2mm of projected distance using an EMS Airflow® prophylaxis master device. This surface is made of pure aluminium (99.5%). The application time is 30 seconds. The plate is put on an elevator to reach the distance of 2 mm. The nozzle is fixed in a resin mould in order to fix well the nozzle position. The mass is known by weighing the powder chamber before and after the test. Every measurement is repeated at least three times and the average is taken as abrasiveness. The depth of the holes was measured by a laser profilometer.

The average particle size of the powders according to the invention was determined in a Malvern Mastersizer 2000 (Malvern Instruments Ltd., Malvern, United Kingdom) with a scirocco dispersing unit at a dispersion pressure of 1.5 bar.

### Example 3: Abrasiveness compared to tagatose and trehalose.

The rhamnose powder according to example 1 was tested for abrasiveness in comparison to tagatose and trehalose powders. The average particle size (d₅₀) of tagatose and trehalose was 14 µm and 24 µm respectively and the maximum particle size (d₁₀₀) of tagatose and trehalose was 80 µm and 600 µm respectively.

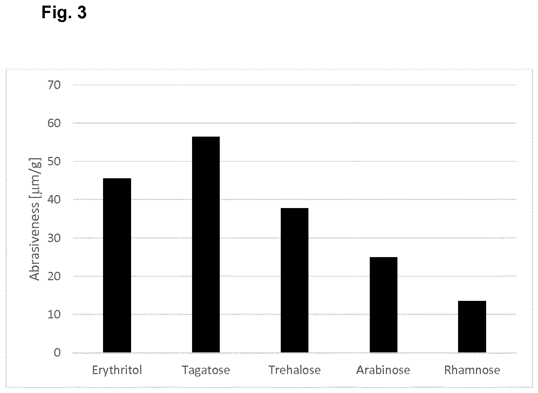

The invention is described further below with reference to Figures 1 to 3.
Fig. 1 shows the cleaning efficiency [mm²/g] of arabinose and rhamnose compared to the commercially available cleaning powders PLUS from EMS (based on erythritol), COMFORT from EMS (based on sodium bicarbonate) and PERIO from EMS (based on glycine).
Fig. 2 shows the abrasiveness [µm/g] of the arabinose and rhamnose powders according to Examples 1 and 2 compared to the commercially available cleaning powders PLUS from EMS (based on erythritol) and COMFORT from EMS (based on sodium bicarbonate).
Fig. 3 shows the abrasiveness of rhamnose in comparison to the PLUS powder, tagatose and trehalose powders according to Example 3.

It has been demonstrated that the powders according to invention comprising rhamnose and arabinose have an improved abrasiveness as well as an improved cleaning efficiency compared to commercially available dental cleaning powders and compared to tagatose and trehalose.

## Claims

1. Use of a powder in a powder jet device for cleaning tooth surfaces by powder spraying, **characterized in that** the powder comprises rhamnose and/or arabinose.

2. Use according to claim 1, **characterized in that** the powder comprises ≥ 50 wt.-% rhamnose and/or arabinose, based on the total weight of the powder.

3. Use according to claim 1 or claim 2, **characterized in that** the powder comprises 60 to 99.5 wt.-% rhamnose and/or arabinose, based on the total weight of the powder.

4. Use according to any of claims 1 to 3, **characterized in that** the powder further comprises 0.5 to 2 wt.-% amorphous silicon dioxide.

5. Use according to any of claims 1 to 4, **characterized in that** the maximum particle size (d₁₀₀) of the powder is ≤ 200 µm.

6. Use according to any of claims 1 to 5, **characterized in that** the maximum particle size (d₁₀₀) of the powder is ≤ 120µm.

7. Use according to any of claims 1 to 6, **characterized in that** the average particle size (d₅₀) of the powder is 5 to 40 µm.

8. Use according to any of claims 1 to 7, **characterized in that** the powder further comprises glycine, sodium hydrogen carbonate and/or erythritol.

9. Use according to any of claims 1 to 8, **characterized in that** the powder further comprises a bactericide, a bleaching agent and/or an analgesic.

10. Process for cleaning tooth surfaces, **characterized in that** a powder according to any of claims 1 to 9 is sprayed with a powder jet device onto a tooth surface together with a gaseous or liquid carrier medium.

11. Powder or powder mixture for use in a powder jet device for cleaning tooth surfaces by powder spraying, **characterized in that** the powder or powder mixture comprises rhamnose and/or arabinose.

12. Powder or powder mixture according to claim 11, **characterized in that** the powder or powder mixture comprises 60 to 99.5 wt.-% rhamnose and/or arabinose, based on the total weight of the powder, 0.5 to 2 wt.- % amorphous silicon dioxide, based on the total weight of the powder, the maximum particle size (d₁₀₀) of the powder is ≤ 120µm and average particle size (d₅₀) of the powder is 5 to 40 µm.
